# EUROPÄISCHE PATENTSCHRIFT

(11) **EP 0 338 444 B1**
(45) Veröffentlichungstag und Bekanntmachung des Hinweises auf die Patenterteilung: **24.11.1993**
(21) Anmeldenummer: 89106685.4
(22) Anmeldetag: 14.04.1989
(51) Int. Cl.: A61K 31/55, A61K 9/18, A61K 9/20, A61K 9/32, A61K 9/36, A61K 9/52

(54) **Azelastin enthaltende Arzneimittel mit kontrollierter Wirkstoffabgabe**
Substituted release azelastine containing medicaments
Médicaments à libération contrôlée contenant de l'azélastine

(30) Priorität: 20.04.1988 DE 3813244
(43) Veröffentlichungstag der Anmeldung: 25.10.1989
(73) Patentinhaber: ASTA Medica Aktiengesellschaft, D-01277 Dresden (DE)
(72) Erfinder: Hettche, Helmut, Dr., D-6057 Dietzenbach (DE)

(56) Entgegenhaltungen:
- EP-A- 0 174 464
- LEHRBUCH DER PHARMAZEUTISCHEN TECHNOLOGIE, 5. Aufl., Verlag Chemie; R. VOIGT, Seite 673#
- PHARMAZEUTISCHE TECHNOLOGIE, 1986, Georg Thieme Verlag, Stuttgart (DE); K.H. BAUER et al.#
- PHARMAZEUTISCHE TECHNOLOGIE, 2. Aufl., 1991; SUCKER et al., Seite 381#

## Beschreibung

Azelastin ist ein Phthalazinon-Derivat folgender Strukturformel: 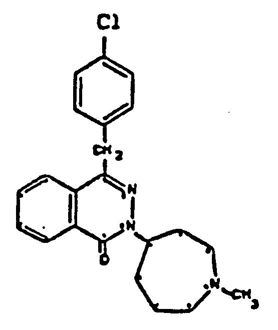
Die chemische Bezeichnung ist: 4-(4-Chlorbenzyl)-2-(perhydro-1-methylazepin-4-yl)-1-(2H)phthalazinon. Azelastin wird insbesondere zur Asthmaprophylaxe eingesetzt. Azelastin hat ebenfalls antiallergische und antihistaminische Eigenschaften, siehe DE-PS 21 64 058.

Einer der wesentlichen Nachteile bei der Anwendung von Azelastin besteht in der Nebenwirkung Müdigkeit. Viele Patienten berichten auch von Schläfrigkeit, Benommenheit und dergleichen. Insbesondere in den ersten Tagen der Anwendung von Azelastin kommt es zur Ausbildung dieser Nebenwirkung, so daß die Bedienung von Kraftfahrzeugen und Maschinen durch den Patienten nicht möglich sowie die allgemeine Aufmerksamkeit dieser Patienten stark reduziert ist.

Es wäre daher höchst wünschenswert und als ein wichtiger medizinischer Fortschritt anzusehen, wenn man über Darreichungsformen des Azelastin verfügen würde, die die Nebenwirkung Müdigkeit nicht aufweisen.

In der Vergangenheit verfuhr man zur Behebung der Nebenwirkung üblicherweise dergestalt, daß man ein Kombinationspräparat aus der betreffenden müdemachenden Wirksubstanz und Coffein entwickelte. Das Coffein hatte dabei die Aufgabe, die sedierende Eigenschaft der Wirksubstanz zu antagonisieren. Dieses Verfahren läßt sich bei Azelastin nicht anwenden, da sich die Eliminationshalbwertszeiten t 1/2 (t1/2 ist die Zeit, innerhalb derer sich der Serumspiegel der Wirksubstanz im Blut ohne weitere Wirkstoffzufuhr von einem bestimmten Ausgangswert auf die Hälfte dieses Wertes erniedrigt) von Azelastin und Coffein stark voneinander unterscheiden: t1/2 von Azelastin beträgt 20 Stunden, t1/2 von Coffein jedoch nur 3,5 Stunden. Somit ist damit zu rechnen, daß bei gleichzeitiger Gabe von Azelastin und Coffein nach einer bestimmten Zeit der Effekt des Coffeins abklingt, wodurch der sedierende Effekt des Azelastins wieder in Erscheinung tritt.

Der übliche Kunstgriff besteht nun darin, in der Darreichungsform die Freisetzung des Wirkstoffs Coffein so stark zu verzögern, daß eine verlängerte Wirkzeit resultiert. Dieses Verfahren ist aber mit der Schwierigkeit verbunden, die Blutspiegelverläufe der beiden Wirksubstanzen in vivo eng aneinander anzugleichen. Dies ist wegen der großen Unterschiede in den Eliminationswerten mit den derzeit zur Verfügung stehenden Techniken nicht möglich.

Das Azelastin besitzt einen außerordentlich Unangenehmen Geschmack, so daß beispielsweise insbesondere flüssige Azelastin-Zubereitungen (zum Beispiel Saft) von den Patienten, insbesondere von Kindern nicht angenommen beziehungsweise verweigert werden. Erfindungsgemäß sind nun aber auch Azelastin-Zubereitungen möglich, deren Geschmack erheblich verbessert ist.

Aufgabe der Erfindung ist es, eine Arzneimittelzubereitung mit dem Wirkstoff Azelastin und ein Verfahren zu ihrer Herstellung zu schaffen, wobei die sedierenden Nebenwirkungen des Azelastins weitgehend beziehungsweise ganz unterdrückt sind.

Die Aufgabe wird gelöst durch eine Arzneimittelzubereitung mit kontrollierter Wirkstofffreigabe, enthaltend übliche Hilfs- und Zusatzstoffe und eine Retardkomponente, die dadurch gekennzeichnet ist, daß der Wirkstoff Azelastin beziehungsweise seine physiologisch verträglichen Salze kontrolliert freigesetzt wird, in einer Menge von 1 Gew.-Teil Azelastin, bezogen auf die Base, zu 0,004 bis 800 Gew.-Teilen Retardkomponente vorhanden ist und die Freisetzung von 0,05 bis 5 mg, beispielsweise 0,05 mg bis 3 mg oder auch 0,05 mg bis 1 mg Azelastin/Stunde beträgt.

Wenn das Azelastin in Form eines Salzes vorliegt, erhöht sich die vorstehend angegebene, sich auf die Base beziehende Azelastinmenge durch das höhere Molekulargewicht des Salzes entsprechend. Die Mengenangaben in der Anmeldung bei Angabe 'Azelastin' beziehen sich stets auf die Base und sind bei Vorliegen eines Salzes entsprechend dem erhöhten Molekulargewicht umzurechnen.

Die Aufgabe schließt auch ein Verfahren ein zur Herstellung einer Arzneimittelzubereitung mit kontrollierter Wirkstofffreigabe durch Einbringen eines Wirkstoffes in übliche Hilfs- und Zusatzstoffe und eine Retardkomponente, das dadurch gekennzeichnet ist, daß man als kontrolliert freizusetzenden Wirkstoff Azelastin bzw. seine physiologisch verträglichen Salze im Verhältnis 1 Gew.-Teil Azelastin, bezogen auf die Base, auf 0,004 bis 800 Gew.-Teile Retardkomponente verwendet und eine Freisetzungsgeschwindigkeit von 0,05 bis 5 mg Azelastin/Stunde einstellt.

In den Unteransprüchen sind bevorzugte Ausführungsformen der Erfindung beschrieben.

Es war überraschend festzustellen, daß es zum Erhalt einer Darreichungsform von Azelastin ohne die Nebenwirkung Müdigkeit gar nicht des Zusatzes von Coffein -ob retardiert oder unretardiert- bedarf, sondern es bereits ausreicht, wenn man den Wirkstoff Azelastin selbst retardiert. Wenn man also den Wirkstoff Azelastin in eine Darreichungsform bringt, die den Wirkstoff über einen längeren Zeitraum freisetzt (und man damit eine 'Retardierung' der Wirksubstanz durchführt), stellt man bei mit dieser Darreichungsform behandelten Personen die Nebenwirkung Müdigkeit nicht mehr fest. Dies ist um so mehr überraschend, als Azelastin eine Substanz mit der bereits erwähnten Eliminationshalbwertszeit von 20 Stunden ist.

In der pharmazeutischen Technologie wandte man bisher das Verfahren der Retardierung nur auf Substanzen mit kurzen Halbwertszeiten an, z.B. von bis zu maximal 10 Stunden. Die Retardierung einer Wirksubstanz, die eine Halbwertszeit von 20 Stunden aufweist, wird bisher in der pharmazeutischen Wissenschaft geradezu als Kunstfehler angesehen.

Überraschend ist bei diesen Darreichungsformen zusätzlich, daß bei deren Applikation der bisher beobachtete bittere Geschmack nach der Einnahme nicht auftritt.

Gegenstand der Erfindung sind also Darreichungsformen mit kontrollierter Abgabe des Wirkstoffes Azelastin bzw. von physiologisch verträglichen Salzen des Azelastin. Geeignete Salze sind beispielsweise Chlorid, Acetat, Maleinat, Lactat, Citrat, Tartrat, Gluconat, Embonat.

Ein weiterer Gegenstand der Erfindung ist ein Verfahren zur Herstellung von Darreichungsformen mit kontrollierter Abgabe des Wirkstoffes Azelastin bzw. von physiologisch verträglichen Salzen des Azelastin.

Die Bestimmung der Freisetzungsgeschwindigkeit des Azelastins zwischen 0,05 und 5 mg pro Stunde wird in einer wässrigen Prüflösung vom pH 1,0 und/oder pH 6,8 bestimmt. Bei dieser Prüflösung handelt es sich um eine wässrige Lösung mit den angegebenen pH-Werten. Die Einstellung der pH-Werte erfolgt durch Zusatz von Säure oder durch Zusatz eines üblichen Puffers.

Als Darreichungsformen kommen zum Beispiel in Frage: Retardtabletten, Retardkapseln, Pellets, Säfte, Implantate, Injektionen, Pflaster, wässrige oder ölige Suspensionen, ölige Lösungen, Granulate, Dragees, Weichgelatinekapseln, Mikrokapseln.

Die erfindungsgemäßen Zubereitungen können wie folgend erhalten werden:
1. Durch Bindung von Azelastin an physiologisch verträgliche Kationenaustauscher. Als solche Kationenaustauscher können z.B. eingesetzt werden:
   Acryl- und Methacrylharze mit austauschbarem Proton, sauren Gruppen: COO^{⊖}, z.B. Amberlite® IRP-64
   Polystyrolharze mit austauschbarem Na⁺, saure Gruppen: SO₃^{⊖}, z.B. Amberlite® IRP-69
   Bei den Ionenaustauschern handelt es sich um saure Ionenaustauscher. Das Maximalverhältnis Azelastin: Ionenaustauscher beträgt etwa 1:1, das Minimalverhältnis etwa 1 Gewichtsteil Wirkstoff auf 800 Teile Ionenaustauscherharz. Vorzugsweise werden auf 1 Gew.-Teil Wirkstoff 1 bis 400 Gew.-Teile Ionenaustauscher, ganz besonders bevorzugt 1 bis 100 Gew.-Teile Ionenaustauscher verwendet.
   Die Bindung des Azelastins erfolgt, indem man eine Azelastinlösung durch ein Bett des Ionenaustauschers in einer Säule laufen läßt oder den Ionenaustauscher in einer Lösung von Azelastin suspendiert, nach Rühren abfiltriert und wäscht. Der beladene Ionenaustauscher wird getrocknet bei Temperaturen bis zu etwa 50^{o}C. Vorzugsweise werden die beladenen Ionenaustauscherpartikel noch mit einer Umhüllung versehen, wie es beispielsweise in US-A-4 221 776 beschrieben ist. Ein Vorteil des zusätzlichen Einhüllens besteht darin, daß sich die Freisetzungsrate des Wirkstoffes durch die Wahl des Hüllenmaterials verändern und beeinflussen läßt. Das Trocknen der mit Hülle versehenen beladenen Ionenaustauscherpartikel kann mit Warmluft von 70^{o}C bis 90^{o}C erfolgen.
   Die beladenen Ionenaustauscherpartikel können in Hartgelatinekapseln gefüllt, oder mit Hilfe von Wasser und Verdickungsmitteln, Geschmacks- und Stabilisierungs- und Konservierungsstoffen eine Suspension als Darreichungsform hergestellt werden.
2. Umhüllung von Wirkstoffteilchen, Granulat- oder Pelletkörner oder Azelastin enthaltende Tabletten mit Überzügen aus folgenden Substanzen, wobei diese Hüllsubstanzen auch in Mischung verwendet werden können: Hydroxypropylmethylcellulosephthalat oder -acetatsuccinat; Cellulose-, Stärke-, sowie Polyvinylacetatphthalat; Carboxymethylcellulose; Polyvinylacetat; Methylcellulose-phthalat, Methylcellulose-succinat, Methylcellulose-phthalatsuccinat sowie Methylcellulose-phthalsäurehalbester; Zein; Ethylcellulose sowie Ethylcellulose-succinat; Schellack; Gluten; Ethylcarboxyethylcellulose; Ethacrylat- Maleinsäureanhydrid-Copolymer; Maleinsäureanhydrid-Vinylmethylether-Copolymer; Styrol-Maleinsäure-Copolymerisate; 2-Ethyl-hexyl-acrylat-maleinsäureanhydrid; Crotonsäure-Vinylacetat-Copolymer; Glutaminsäure/Glutaminsäureester-Copolymer; Carboxymethylethylcelluloseglycerinmonooctanoat; Celluloseacetatsuccinat; Polyarginin; Fette, Öle, Wachse, Fettalkohole; Anionische Polymerisate aus Methacrylsäure und Methacrylsäureestern (Eudragit®L, Eudragit®S); Copolymerisate aus Acryl- und Methacrylsäureestern mit einem geringen Gehalt an Ammoniumgruppen (Eudragit®RS), sowie Copolymere aus Acryl- und Methacrylsäureestern und Trimethylammoniummethacrylat (Eudragit®RL), Copolymerisat aus Acrylsäureäthyl- und Methacrylsäuremethylestern 70:30 (Eudragit®NE 30 D), Copolymerisat aus Acrylsäure, Methacrylsäure sowie deren Estern (Verhältnis der freien Carboxylgruppen zu den Estergruppen z.B. 1:1) (Eudragit®L 30 D).
   Die genannten Substanzen können zusätzlich übliche Weichmacher (z.B. Dibutylsebacat, Citronen- und Weinsäureester, Glycerin und Glycerinester, Phthalsäureester und ähnliche Stoffe) enthalten, außerdem den Zusatz wasserlöslicher Substanzen wie Polyethylenglykole, Polyvinylpyrrolidon, Copolymerisat aus Polyvinylpyrrolidon und Polyvinylacetat, Hydroxypropylcellulose, Hydroxypropylmethylcellulose. Auch der Zusatz von Feststoffen wie Talkum und/oder Magnesiumstearat in der Umhüllung ist möglich.
   Die Pelletkörner, Granulatkörner oder Tabletten können auch Zusätze von organischen Säuren (wie z.B. Citronensäure, Weinsäure, Malein-, Fumar-, Ascorbinsäure) eingearbeitet enthalten.
   Die Umhüllung erfolgt durch Aufsprühen von Lösungen in organischen Lösungsmitteln oder Suspensionen der genannten Substanzen in organischen Lösungsmitteln oder Wasser, wobei noch weitere Hilfsstoffe zur Optimierung deren Verarbeitbarkeit zugesetzt sein können, wie z.B. oberflächenaktive Substanzen, Pigmente.
   Das Aufsprühen erfolgt z.B. im Dragierkessel oder in perforierten Kesseln, oder im Luftsuspensionsverfahren (z.B. Glatt Wirbelschichtanlage WLSD5).
   Die Umhüllung kann auch im Koazervationsverfahren erfolgen, wobei sogenannte Mikrokapseln gebildet werden.
   Die Umhüllung kann auch durch Koagulation wässriger Dispersionen der zuvor genannten Substanzen erfolgen, indem der Wirkstoff mit der Dispersion vermischt und das Wasser durch Trocknen entfernt wird.
   Überzogene Wirkstoffteilchen und überzogene Granulate können zu Tabletten verpreßt werden, überzogene Pellets in Hartgelatinekapseln abgefüllt werden.
   Beim Überziehen von Wirkstoffteilchen oder Granulaten, die Wirkstoffteilchen enthalten, wird überlicherweise mehr Hüllstoff eingesetzt als bei Pellets, da die Oberfläche, die bedeckt werden muß, wesentlich größer ist als bei Pellets.
   Auf 1 Gew.-Teil Wirkstoff können 0,004 bis 800 Gew.-Teile Hüllstoff verwendet werden. Bevorzugt ist ein Gewichtsverhältnis von 1 Teil Wirkstoff und 0,005 bis 500 Gew.-Teilen Hüllmaterial, ganz besonders bevorzugt sind 0,01 bis 200 Gew.-Teile Hüllmaterial pro 1 Gew.-Teil Wirkstoff. Das Aufbringen der Hüllstoffe erfolgt bei erhöhter Temperatur, vorzugsweise im Luftstrom. Zulufttemperatur z. B. 70 bis 90^{o} C; Temperatur der Abluft z. B. bis 40^{o} C.
3. Umhüllung von Preßlingen, Tabletten, Granulaten, die Azelastin und eine oder mehrere osmotisch aktive Substanzen (z.B. Mannit, Sorbit) enthalten mit einer semi-permeablen Membran, z.B. aus 70 bis 90 Gewichts% Celluloseacetat und Hydroxypropylmethylcellulose (30 bis 10 Gewichts%).
   Als osmotisch aktive Substanzen kommen weiterhin in Frage: organische und anorganische Verbindungen oder lösliche Stoffe, die einen osmotischen Druckgradienten gegenüber der äußeren Flüssigkeit über die semipermeable Wand erzeugen. Osmotisch wirksame Mittel oder osmotisch wirksame Verbindungen umfassen Magnesiumsulfat, Magnesiumchlorid, Natriumchlorid, Lithiumchlorid, Kaliumsulfat , Kaliumhydrogenphosphat, Harnstoff, Saccharose und ähnliches. Weitere osmotisch wirksame Mittel sind bekannt aus den US-PS 3 854 770, 4 077 407 und 4 235 236.
   Als semi-permeable Materialien, die als Polymere zur Osmose und umgekehrten Osmose bekannt sind, kommen z. B. in Frage: Cellulose-acylat, Cellulose-diacylat, Cellulose-triacylat, Cellulose-acetat, Cellulose-diacetat, Cellulose-triacetat, β-Glucan-acetat, Acetaldehyd-dimethyl-acetat, Cellulose-acetat-ethyl-carbamat, Polyamid, Polyurethan, sulfoniertes Polystyrol, Cellulose-acetat-phthalat, Cellulose-acetatmethyl-carbamat, Cellulose-acetat-succinat, Cellulose-acetat-dimethylaminoacetat, Cellulose-acetat-chloracetat, Cellulose-dipalmitat, Cellulose-dioctanoat, Cellulose-dicaprylat, Cellulose-dipentanat, Cellulose-acetat-valerat, Cellulose-acetat-p-toluolsulfonat, Cellulose-acetat-butyrat, Ethylcellulose, selektiv permeable Polymere, die gebildet worden sind durch gemeinsame Ausfällung eines Polykations und eines Polyanions wie in den US- 3,173,876, 3,276,586, 3,541,005, 3,541,006 und 3,546,142 angegeben. Derartige Einhüllungen in semi-permeable Membranen können z. B. auch gemäß DE-A-33 10 081 oder DE-A-33 10 096 erfolgen.
   Der Anteil an osmotisch aktiver Substanz kann, bezogen auf 1 Gew.-Teil Azelastin, von 10 bis 800 Gew.-Teilen, vorzugsweise 20 bis 600, ganz besonders bevorzugt 50 bis 400 Gew.-Teilen betragen. Die Hüllstoffe werden in einer Menge aufgebracht, daß die semipermeable Membran 50 bis 500 µm, vorzugsweise 100 bis 300 µm dick ist.
   Das Verarbeiten des Wirkstoffes und der osmotisch aktiven Substanzen kann zwischen Raumtemperatur und 80°C erfolgen. Zur Einstellung der Freisetzungsrate wird zum Beispiel mit Hilfe eines Laserstrahls ein Loch in die Membranwand gebohrt, so daß nach dem Einbringen der daraus hergestellten Tabletten in wässrige Flüssigkeit der Wirkstoff durch einwandernde Flüssigkeit gelöst bzw. suspendiert und durch das Loch herausgedrückt wird. Das Aufbringen der semipermeablen Schicht erfolgt zum Beispiel bei 70 - 90^{o} C Zulufttemperatur.
   Gegebenenfalls kann die semipermeable Membran auch noch eine mikroporöse Schicht enthalten beziehungsweise können mikroporöse Stoffe eingearbeitet sein (siehe hierzu Deutsche Offenlegungsschrift 33 10 081, zum Beispiel Seite 7 - 17).
   Materialien, die zur Herstellung der mikroporösen Schicht geeignet sind, umfassen zum Beispiel Polycarbonate aus linearen Polyestern der Kohlensäure, in denen Carbonatgruppen in der Polymerkette wiederkehren, mikroporöse Materialien, die hergestellt worden sind durch Phosgenierung einer dihydroxy-aromatischen Verbindung wie Bisphenol, einem mikroporösen Polyvinylchlorid, mikroporösen Polyamiden wie Polyhexamethylen-adipinsäureamid, mikroporöse Modacrylpolymere einschließlich solchen, die gebildet worden sind aus Polyvinylchlorid und Acrylnitril, mikroporöse Styrol-Acrylmonomer und deren Copolymere, poröse Polysulfone charakterisiert durch Diphenylensulfon in einer linearen Kette, halogeniertes Polyvinyliden, Polychlorether, Acetalpolymere, Polyester hergestellt durch Veresterung einer Dicarbonsäure oder eines Anhydrids mit einem Alkylenpolyol, Polyalkylensulfide, phenolische Polymere, Polyester, mikroporöse Polysaccharide mit substituierten Anhydroglucoseeinheiten, die eine abnehmende Durchlässigkeit für Wasser und biologische Flüssigkeiten besitzen, asymmetrische poröse Polymere, vernetzte Olefinpolymere, hydrophobe oder hydrophile mikroporöse Homopolymere, Copolymere oder Interpolymere mit einer verringerten Dichte sowie Materialen, die beschrieben sind in den
   US-PS 3 595 752, 3 643 178, 3 654 066, 3 709 774, 3 718 532, 3 803 601, 3 852 224, 3 852 388 und 3 853 601; in der GB-PS 1 126 849 und in Chemical Abstracts Band 71, 427F, 22573F, 1969.
   Weitere mikroporöse Materialien zur Herstellung der mikroporösen Schicht umfassen Polyurethane, vernetzte kettenverlängerte Polyurethane, Polyimide, Polybenzimidazole, Collodium, regenerierte Proteine, halbfestes vernetztes Polyvinyl-pyrrolidon, mikroporöse Materialien, die hergestellt worden sind durch Diffusion von mehrwertigen Kationen in Polyelektrolytsole, mikroporöse Derivate von Polystyrol wie Natriumpolystyrol-sulfonat, Polyvinylbenzyl-trimethyl-ammonium-chlorid, mikroporöse Cellulose-acylate und ähnliche mikroporöse Polymere sind bekannt aus den US-PS 3 524 753, 3 565 259, 3 276 589, 3 541 055, 3 541 006, 3 546 142, 3 615 024, 3 646 178 und 3 852 224.
   Die zur Herstellung der mikroporösen Schicht geeigneten Porenbildner, umfassen Feststoffe und porenbildende Flüssigkeiten. Der Ausdruck Porenbildner, wie er hier verwendet wird, umfaßt auch Substanzen, die Mikrodurchgänge bilden und die Entfernung der Porenbildner kann zu beiden Arten führen. Der Ausdruck porenbildende Flüssigkeiten umfaßt im Rahmen dieser Beschreibung halbfeste und viskose Flüssigkeiten. Die Porenbildner können anorganisch oder organisch sein und das schichtbildende Polymer enthält im allgemeinen 5 bis 70 Gewichts% Porenbildner, insbesondere 20 bis 50 Gewichts%. Der Ausdruck Porenbildner sowohl für Feststoffe als auch für Flüssigkeiten umfaßt Substanzen, die durch die in der Anwendungsumgebung vorhandene Flüssigkeit aus dem Vorläufer der mikroporösen Membran herausgelöst, extrahiert oder ausgelaugt werden können, unter Bildung einer wirksamen offenzelligen mikroporösen Schicht. Die porenbildenden Feststoffe haben eine Teilchengröße von ungefähr 0,1 bis 200 µm und umfassen Alkalisalze wie Lithiumcarbonat, Natriumchlorid, Natriumbromid, Kaliumchlorid, Kaliumsulfat, Kaliumphosphat, Natriumacetat, Natriumcitrat und ähnliches. Organische Verbindungen wie Saccharide einschließlich der Zucker Saccharose, Glucose, Fructose, Mannit, Mannose, Galactose, Sorbit und ähnliches. Es können auch lösliche Polymere zur Anwendung kommen wie Carbowaxe, Carbopol und ähnliches. Die Porenbildner umfassen ferner Diole, Polyole, mehrwertige Alkohole, Polyalkylenglykole, Polyglykole, Poly(α-ω)-alkylendiole und ähnliches.
4. Einbettung von Azelastin Wirkstoff oder Bindung an folgende Substanzen oder Mischungen dieser Substanzen:
   - Verdauliche Fette, z.B.
      Triglyceride von gesättigten Fettsäuren C₈H₁₆O₂ bis C₁₈H₃₆O₂ und deren Gemische, Erdnußöl und hydriertes Erdnußöl, Ricinusöl und hydriertes Ricinusöl, Olivenöl, Sesamöl, Baumwollsamenöl und hydriertes Baumwollsamenöl, Maisöl, Weizenkeimöl, Sonnenblumensamenöl, Kabeljau-Leberöl, Gemische von Mono-, Di-, Triestern der Palmitin- und Stearinsäure mit Glycerin, Glycerintrioleat, Diglykolstearat, Stearinsäure.
   - Unverdauliche Fette bzw. fettähnliche Substanzen, z.B. Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome), Carnaubawachs, Bienenwachs, Fettalkohole (geradkettig oder verzweigtkettig) der Kettenlänge C₈H₁₇OH bis C₃₀H₆₁OH, insbesondere C₁₂H₂₅OH bis C₂₄H₄₉OH.
   - Polymere wie
      Polyvinylalkohol, Polyvinylchlorid, Polyacrylsäure (Carbopol®); anionische Polymerisate aus Methacrylsäure und Methacrylsäureestern (Eudragit®L, Eudragit®S), Acryl- und Methacrylsäureester-Copolymerisate mit Trimethylammoniummethacrylat (Eudragit®RL, Eudragit®RS)
   Copolymerisat von Acrylsäureethyl- und Methacrylsäuremethylestern (Eudragit®NE 30 D), sowie aus Acrylsäure, Methacrylsäure sowie deren Estern (Verhältnis der freien Carboxylgruppen zu den Estergruppen 1:1) (Eudragit® -L 30 D), Polyethylen, Polyglycolsäure, Polyhydroxybuttersäure, Polymilchsäure, Copolymere aus Milchsäure und Glycolsäure (Hersteller: Boehringer Ingelheim), Copolymere aus Milchsäure und Ethylenoxid, Copolymere aus Glycolsäure und Ethylenoxid, Copolymere aus Milchsäure und Hydroxybuttersäure, Hydroxypropylmethylcellulose-phthalat oder acetatsuccinat; Celluloseacetatphthalat, Stärkeacetatphthalat, sowie Polyvinylacetatphthalat; Carboxymethylcellulose; Methylcellulose-phthalat, -succinat, -phthalatsuccinat, Methylcellulose-phthalsäurehalbester; Zein; Ethylcellulose; Schellack, Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethylhexyl-acrylat-maleinsäureanhydrid; Crotonsäure-Vinylacetat-Copolymer; Glutaminsäure/Glutaminsäureester-Copolymer; Carboxmethylcellulose-glycerinmonooctanoat; Celluloseacetatsuccinat; Polyarginin; quervernetztes Alginat; quervernetzte Gelatine;
   - Quellstoffe wie Methylcellulose, Hydroxypropylcellulose, Hydroxypropylmethylcellulose (Pharmacoat, Methocel E = Propylenglykoläther der Methylcellulose), Alginsäure und ihre Salze (Na-, Ca-Salz, auch Mischungen aus Natriumalginat und Calciumsalzen z.B. CaHPO₄), Stärke, Carboxymethylstärke, Carboxymethylcellulose und deren Salze (z. B. Na-Salz), Galaktomannan, Gummi arabicum, Karaya Gummi, Ghatti Gum, Agar-agar, Carrageen, Xanthan-Gummi, Guar Gummi und seine Derivate, Johannisbrotkernmehl, Propylenglykolalginat, Pektin, Traganth.

Auf 1 Gew.-Teil Azelastin werden bei diesen Retardkomponenten 1 bis 800 Gew.-Teile Retardkomponente, vorzugsweise 1,5 bis 600 Gew.-Teile, ganz besonders bevorzugt 2,0 bis 400 Gew.-Teile verwendet. Die Herstellung dieser Zubereitungen erfolgt bei Temperaturen zwischen 18^{°}C und 80^{°}C.

Die Herstellung dieser Darreichungsform kann erfolgen:
a) durch Lösen oder Dispergieren von Azelastin oder seinen Salzen in den genannten Fetten oder fettähnlichen Substanzen oder Mischungen davon, auch unter Schmelzen der genannten Substanzen und anschließend Wiederabkühlen, Zerkleinern, evtl. Zufügen weiterer Substanzen wie z.B. die oben erwähnten wasserlöslichen oder in Wasser quellbaren Substanzen und Verpressung zu Tabletten. Das Abkühlen der Schmelze und Zerkleinern kann auch in einem Schritt zusammengefasst werden, indem die Schmelze in kaltem Wasser dispergiert wird oder einer Spüherstarrung unterworfen wird.
   Bei Verwendung der obengenannten Öle als Retardierungsmittel wird Azelastin bzw. dessen Salz im Öl gelöst oder suspendiert und, evtl. nach Zusatz von bis zu 2 % Aluminiummonostearat, in Ampullen abgefüllt und nachfolgend sterilisiert oder, evtl. nach Zusatz von Geschmacksstoffen und/oder Sedimentationsverzögerern, wie hochdisperses Siliciumdioxid (z.B. Aerosil®) homogenisiert und in Flaschen gefüllt,
b) durch Mischen von Azelastin mit den genannten Fetten, Polymeren oder Quellstoffen oder Mischungen dieser Substanzen, auch unter Anwendung von Wärme, und Verpressen der Mischungen, evtl. nach Zusatz weiterer Hilfsstoffe, zu Tabletten oder Formung zu Pellets,
c) durch Mischen von Azelastin mit Lösungen der genannten Fette oder Polymeren in Wasser oder organischen Lösungsmitteln, wie z.B. Ethanol, Ethylacetat, Aceton oder Isopropanol, evtl. Mischen mit Trägermaterialien wie Cellulosen, sowie nachfolgendes Abdampfen der Lösungsmittel und Vermischen der erhaltenen Wirkstoffeinbettung mit weiteren Hilfsstoffen und Verarbeitung zu Formkörpern, wie z.B. Tabletten oder Pellets,
d) durch Anfeuchten einer Mischung aus Azelastin und den genannten Quellstoffen mit organischen Lösungsmitteln wie Ethanol, Ethylacetat, Aceton oder Isopropanol, evtl. unter Beifügung von Bindemitteln, wie Polyvinylpyrrolidon oder Copolymeren aus Polyvinylpyrrolidon und Polyvinylacetat, Granulieren der erhaltenen Mischung, nachfolgendes Trocknen, Zusatz von evtl. weiteren Hilfsstoffen und Pressung der Mischung zu Tabletten,
e) durch Mischen von Azelastin mit einer Lösung von Natur-oder Kunstharzen, wie Schellack oder Polyvinylacetet in Polyethylenglykol vom Molgewicht 200 bis 1500, evtl. Zusatz weiterer Hilfsstoffe, wie z.B. Stearate oder Quellstoffe und Verkapseln der erhaltenen Masse in Weich- oder Hartgelatinekapseln.

Ganz allgemein erfolgt die Herstellung der Arzneimittelzubereitungen in an sich bekannter Weise, wobei neben den Retardkomponenten die bekannten und üblichen pharmazeutischen Hilfsstoffe sowie sonstige übliche Träger- und Verdünnungsmittel verwendet werden können, wobei die als Retardkomponente genannten Hilfsstoffe auch noch andere Funktionen ausüben können, z.B. als Formtrennmittel oder als Sprengmittel wirken.
Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u. ff., H.v. Czetsch-Lindenwald, Hilfsstoffe für Pharmazie und angrenzende Gebiete; Pharm. Ind., Heft 2, 1961, Seite 72 u. ff.; Dr. H. P. Fiedler, Lexikon der Hilfsstoffe für Pharmazie, Kosmetik und angrenzende Gebiete, 2. Auflage, Editio Cantor, Aulendorf in Württemberg 1981.

Beispiele für übliche Hilfsstoffe, Trägerstoffe und Verdünnungsmittel sind Gelatine, natürliche Zucker, wie Rohrzucker oder Milchzucker, Lecithin, Pektin, Stärke (z.B. Maisstärke) sowie Stärkederivate, Cyclodextrine und Cyclodextrinderivate, Polyvinylpyrrolidon, Gelatine, Gummi arabicum, Alginsäure, Tylose, Talkum, Lycopodium, Kieselsäure (z.B. kolloidale), Lävulose, Traganth, Natriumchlorid, Stearate, Magnesium- und Calciumsalze von Fettsäuren mit 12 bis 22 C-Atomen, insbesondere der gesättigten (z.B. Stearate), Polyethylenglycol mit einem mittleren Molekulargewicht zwischen 200 und 20,000, vorzugsweise zwischen 200 und 5.000, insbesondere zwischen 200 und 1.000, oder deren Gemische und/oder Polymerisate aus Vinylpyrrolidon und/oder Mischpolymerisate aus Vinylpyrrolidon und Vinylacetat, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglycol, Pentaerythrit, Sorbit, Mannit usw., die ggfs. auch verethert sein können, Benzylbenzoat, Dioxolane, Glyzerinformale, Tetrahydrofurfurylalkohol, Polyglykolether mit C₁ bis C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat, Gummi arabicum, Alginsäure, Stearate, Fette und ähnlich wirkende Stoffe.

Daneben können die Darreichungsformen grenzflächenaktive Substanzen enthalten. Als Beispiele seien genannt: Alkaliseifen, wie Alkalisalze höherer Fettsäuren (z.B. Na-palmitat, Na-stearat) oder deren Derivate (z.B.Na-rizinolatschwefelsäureester); sulfurierte Verbindungen oder sulfonierte Verbindungen, die durch Umsetzung von höheren Fettalkoholen mit Schwefelsäure oder Chlorsulfonsäure entstehen und z.B. als Natriumsalze eingesetzt werden (z.B. Natriumlaurylsulfat, Natriumcetylsulfat, Natriumstearylsulfat, Natriumcetylsulfonat); Salze der Gallensäuren; Saponine; quartäre Ammoniumverbindungen; Partialfettsäureester des Sorbitans; Partialfettsäureester und Fettsäureester des Polyoxyethylensorbitans; Sorbitolether des Polyoxyethylens; Fettsäureester des Polyoxyethylens; Fettalkoholether des Polyoxyethylens; Fettsäureester der Saccharose; Fettsäureester des Polyglycerols; Proteine; Lecithine.

Die Darreichungsformen können auch Cellulosen enthalten, insbesondere wenn Komprimate hergestellt werden sollen. Als solche kommen in Frage:
gereinigte Cellulose (z.B. als Elcema® im Handel) oder mikrokristalline Cellulose, wie sie z.B. unter dem Namen Avicel® im Handel ist. Aber auch andere Füllmittel mit Bindemittelfunktion, wie Calciumhydrogenphosphat, Milchzucker, Stärken (z.B. Kartoffelstärke, Maisstärke, modifizierte, Stärken wie Starch ST 1500/Colorcon), Glucose, Mannit, Saccharose können eingesetzt werden.

Die Darreichungsformen können darüber hinaus Sedimentationsverzögerer enthalten, wie z.B. hochdisperse Kieselsäuren, die eine Oberfläche von 50 bis 500 m²/g, insbesondere 100 bis 400 m²/g aufweisen (bestimmt nach der BET-Methode). Diese sind im Handel z.B. unter dem Namen Aerosil® erhältlich.

Außerdem kann der Einsatz von Formtrennmitteln in der Darreichungsform sinnvoll sein. Als solche wären zu nennen:
Talk oder silikonisierter Talk, Calcium- und Magnesiumstearat, Stearinsäure, Paraffin, hydrierte Fette und Öle, Siliconölemulsion.

Als weitere Hilfsstoffe kommen auch Stoffe in Frage, die den Zerfall bewirken (sogenannte Sprengmittel) wie:
quervernetztes Polyvinylpyrrolidon, Natriumcarboxymethylstärke, Natriumcarboxymethylcellulose, Formaldehydgelatine, Formaldehydcasein, Polyacrylsäure und Ultraamylopektin.

Zur Herstellung von Lösungen und Suspensionen kommen beispielsweise Wasser oder physiologisch verträgliche organische Lösungsmittel in Frage, wie z.B. Ethanol, 1,2-Propylenglykol, Polyglykole und deren Derivate. Für injizierbare Lösungen oder Suspensionen kommen zum Beispiel nicht-toxische parenteral verträgliche Verdünnungsmittel oder Lösungsmittel in Frage, wie z.B.:
Wasser, 1,3-Butandiol, Ethanol, 1,2-Propylenglykol, Polyglykole in Mischung mit Wasser, Ringer's Lösung, isotonische Kochsalzlösung.

Darüberhinaus ist der Zusatz von Stabilisatoren, Farbstoffen, Antioxidantien und Komplexbildnern (z.B. Ethylendiaminotetraessigsäure) und dergl. möglich sowie den Zusatz von Säuren wie Citronensäure, Weinsäure, Maleinsäure, Fumarsäure.

Als Antioxidantien kommen beispielsweise Natriummetabisulfit, Cystein, Ascorbinsäure und deren Ester (z.B. -palmitat), Flavonoide, Gallussäure, Gallussäure-alkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Citronensäure, Phosphorsäure) zur Anwendung.

Als Konservierungsmittel kommen beispielsweise Sorbinsäure, p-Hydroxybenzoesäureester (z.B. Niederalkylester), Benzoesäure, Natriumbenzoat, Trichlorisobutylalkohol, Phenol, Kresol, Benzethoniumchlorid und Formalinderivate in Betracht.

Als Plastifizierungsmittel für Hüllstoffe können in Frage kommen:
Citronen- und Weinsäureester (Acetyltriethyl-, Acetyl-tributyl-, Tributyl-, Triethyl-citrat);
Glycerin und Glycerinester (Glycerindiacetat, -triacetat, acetylierte Monoglyceride, Rizinusöl);
Phthalsäureester (Dibutyl-, Diamyl-, Diethyl-, Dimethyl-, Dipropyl-, D-(2-Methoxy- oder ethoxyethyl)-phthalat, Ethylphthalyl- und Butylphthalylethyl- und butylglycolat);
Alkohole (Propylenglykol, Polyethylenglykol verschiedener Kettenlängen), Adipate (Diethyl-, Di(2-Methoxy- oder ethoxyethyl)adipat); Benzophenon; Diethyl- und Dibutylsebacat, -succinat, -tartrat;
Diethylenglykoldipropionat; Ethylenglykoldiacetat, -dibutyrat, -dipropionat; Tributylphosphat, Tributyrin; Polyethylenglykolsorbitanmonooleat; Sorbitanmonooleat.

Zum Aufbringen der Retardkomponenten bzw. Hüllstoffe können Lösungsmittel verwendet werden aus der Gruppe der wässrigen Lösungsmittel, Alkohole, Ketone, Ester, Ether, aliphatischen Kohlenstoffe, halogenierten Lösungsmittel, cycloaliphatischen, aromatischen, heterocyclischen Lösungsmittel und deren Gemische. Typische Lösungsmittel sind unter anderem Aceton, Diaceton-alkohol, Methanol, Ethanol, Isopropyl-alkohol, Butyl-alkohol, Methylacetat, Ethylacetat, Isopropylacetat, n-Butylacetat, Methylisobutyl-keton, Methyl-propylketon, n-Hexan, n-Heptan, Ethyl-glykol-monoethyl-ether, Ethylen-glykol-monoethylacetat, Methylen-dichlorid, Ethylen-dichlorid, Propylen-dichlorid, Tetrachlorkohlenstoff, Nitroethan, Nitropropan, Tetrachlorethan, Ethyl-ether, Isopropyl-ether, Cyclohexan, Cyclo-octan, Benzol, Toluol, Naphtha, 1,4-Dioxan, Tetrahydrofuran, Diethylenglykoldimethylether, Wasser und deren Gemische wie Aceton und Wasser, Aceton und Methanol, Aceton und Ethylalkohol, Methylen-dichlorid und Methanol und Ethylen-dichlorid und Methanol sowie deren Gemische. Im Laufe des Umhüllungsprozesses werden diese Lösungsmittel wieder entfernt. Unabhängig vom Herstellverfahren sind die erfindungsgemäßen Darreichungsformen dadurch gekennzeichnet, daß sie den Wirkstoff Azelastin oder seine physiologisch verträglichen Salze mit einer Freisetzungsgeschwindigkeit zwischen 0,05 und 5 mg pro Stunde an Körperflüssigkeiten abgeben bzw. in diese übertreten lassen.

Dosierungsangaben beziehen sich immer auf Azelastin als Base; werden Salze des Azelastins eingesetzt, so ist entsprechend dem Molgewicht umzurechnen.

Die Gehalte an Azelastinwirkstoff in den erfindungsgemäßen Zubereitungen betragen
a) bei oral anzuwendenden Darreichungsformen
   0,1 mg bis 50 mg, vorzugsweise
   0,2 mg bis 30 mg, inbesondere
   0,5 mg bis 20 mg Azelastin-Wirkstoff.
   Die genannten Einzeldosen können 1- bis 5mal, vorzugsweise 1- bis 3mal, insbesondere 1- bis 2mal täglich angewendet werden,
b) bei parenteral anzuwendenden Darreichungsformen (intravenös, intramuskulär, subcutan, intraperitoneal):
   0,1 mg bis 500 mg, vorzugsweise
   0,2 mg bis 400 mg, insbesondere
   0,5 bis 250 mg Azelastin-Wirkstoff.
   Die genannten Einzeldosen können 1mal monatlich (z.B. bei subcutan zu applizierenden Implantaten) bis 3mal täglich, vorzugsweise 1mal monatlich bis 2mal täglich, insbesondere 1mal monatlich bis 1mal täglich verabreicht werden.
c) bei dermal anzuwendenden Darreichungsformen (z.B. Pflaster)
   5 mg bis 5000 mg, vorzugsweise
   10 mg bis 3000 mg, insbesondere
   30 mg bis 2000 mg Azelastin-Wirkstoff.
   Die genannten Einzeldosen können 1mal täglich bis 1mal monatlich, vorzugsweise 1mal alle 3 Tage bis 1mal alle 3 Wochen, insbesondere wöchentlich bis alle 2 Wochen verabreicht werden.

Besonders bevorzugte Retardkomponenten sind:
a) Kationenaustauscher
   Poly(styrol, divinylbenzol)sulfonsäure-Natrium (z.B. Amberlite® IRP 69).Auf 1 Teil Azelastin (Base) werden z.B. 3 bis 10 Teile Amberlite® IRP 69 eingesetzt.
b) Hüllstoffe
   Hydroxypropylmethylcellulosephthalat
   Auf 1 Teil Azelastin 1,5 bis 3 Teile Hydroxypropylmethylcellulosephthalat 55.
   Ethylcellulose
   Auf 1 Teil Azelastin 0,1 bis 1 Teil Ethylcellulose.
   Eudragitharze wie z.B. Eudragit®RS
   Auf 1 Teil Azelastin 0,01 bis 0,1 Teile Eudragit®RS.
c) Semi-permeable Schichten mit osmotisch wirksamem wirkstoffhaltigen Kern und Austrittsöffnung:
   Umhüllung mit 100 bis 300 µm dicker Schicht aus 82 % Celluloseacetat und 18 % Hydroxypropylmethylcellulose.
d) Einbettungssubstanzen
   Hydrokolloide, z.B. Hydroxypropylmethylcellulose:
   Auf 1 Teil Azelastin 2 bis 10 Teile Hydrokolloid.
   Eudragit®RS:
   Auf 1 Teil Azelastin 10 bis 15 Teile Eudragit®RS .
   Glycerinditripalmitostearat (z.B. Precirol Ato 5)
   Auf 1 Teil Azelastin 1 bis 10 Teile Precirol Ato 5.

Die geforderte Wirkstofffreisetzung von 0,05 bis 5 mg pro Stunde stellt sich durch die Maßnahmen des Verfahrensanspruchs in Verbindung mit den entsprechenden Angaben in der Beschreibung in dem gewünschten Bereich ein. Falls innerhalb dieses Bereichs eine bestimmte Freisetzungsgeschwindigkeit erzielt werden soll, kann man zum Beispiel wie folgt vorgehen:
1. Die Herstellung der Umhüllung beziehungsweise Einbettung der Wirksubstanz auf die beschriebene Art und Weise.
2. Prüfung der Wirkstofffreisetzung aus der Darreichungsform unter Verwendung von 0,1 N HCl (2 Stunden) und Phosphatpuffer pH 6,8 (anschließend) als Freisetzungsmedium.
3.
   a) Erweist sich die Freisetzung als zu hoch:
      Erhöhung des Anteils an Retardkomponente und/oder Erniedrigung des Anteils an wasserlöslichen Hilfsstoffen. Erniedrigung des Anteils an osmotisch aktiver Substanz
   b) Erweist sich die Freisetzung als zu niedrig:
      Erniedrigung des Anteils an Retardkomponente und/oder Erhöhung des Anteils an wasserlöslichen Hilfsstoffen. Erhöhung des Anteils an osmotisch aktiver Substanz

Im allgemeinen wird eine Freisetzungsgeschwindigkeit von 1 mg Azelastin pro Stunde angestrebt.

### Beispiel 1:

100 g Azelastinhydrochlorid werden mit 960 g Hydroxypropylmethylcellulose (Viskosität einer 2 % igen wässrigen Lösung: 4000 mPa · s (4000 cP) (Handelsprodukt: zum Beispiel Methocel K4M Premium)), 1320 g sprühgetrockneter Lactose und 20 g Magnesiumstearat gemischt und die Mischung zu Tabletten vom Gewicht 120 mg, einem Durchmesser von 6 mm und einem wölbungsradius von 6 mm verpreßt.

Im Anschluß daran können die Tabletten nach üblichem Verfahren mit einem magensaftlöslichen oder magensaftpermeablen beziehungsweise magensaftresistenten Filmüberzug versehen werden.

Zur Erzeugung eines magensaftresistenten Überzugs werden 1000 g Tabletten mit etwa 1000 g der folgenden Suspension zum Beispiel in einem Dragierkessel besprüht:
In 480 g Aceton werden 63 g Celluloseacetatphthalat gelöst. In diese Lösung werden 21 g Phthalsäurediethylester, 30 g Dichlormethan und 131 g Methanol eingegeben. In der erhaltenen Lösung werden 4,4 g Titandioxid homogen suspendiert.

Das Sprühen erfolgt diskontinuierlich, wobei zwischen den Sprühphasen erwärmte Trocknungsluft eingeblasen wird.

Eine Retardtablette enthält 5 mg Azelastinhydrochlorid.

### Beispiel 2:

12 g Azelastinhydrochlorid, 20 g Eudragit®RS PM 250 g Talkum und 200 g Lactose werden gemischt und die Mischung mit etwa 140 g einer Mischung von 12,7 g Glycerintriacetat (Handelsbezeichnung zum Beispiel Triacetin) und 127,3 g Eudragit® RS 12,5 angefeuchtet. Die feuchte Masse wird durch ein Sieb der Maschenweite 1 mm in üblicher Weise granuliert und nach dem Trocknen bei Zimmertemperatur im Dragierkessel eine Mischung aus 909 g Eudragit® RS 12,5 und 91 g Triacetin mittels Sprühpistole aufgesprüht. Das erhaltene getrocknete Granulat wird ohne Zusatz weiterer Hilfsstoffe zu bikonvexen Tabletten vom Gewicht 300 mg und einem Durchmesser von 10 mm verpreßt.

Eine Tablette enthält 5 mg Azelastinhydrochlorid in Retardzubereitung.

### Beispiel 3:

50 g Azelastin HCl werden mit 100 g Weinsäure, 250 g Lactose, 10 g mikrokristalliner Cellulose (Avicel PH 101) und 7 g Hydroxypropylcellulose (Viskosität der 5%igen Lösung: 75 bis 150 mPa · s (75 bis 150 cPs (zum Beispiel Handelsname: Klucel LF)) angeteigt. Die feuchte Masse wird durch ein Lochblech mit einem Lochdurchmesser von 1 mm gepreßt und die entstehenden Stränge durch Behandlung auf einer Spheronizer-Scheibe in üblicher Weise zerteilt und ausgerundet. Die erhaltenen Pellets werden getrocknet und gesiebt. 300 g Pellets der Siebfraktion 800 bis 1200 um werden mit einer Lösung aus 42,5 g Ethylcellulose (Handelsbezeichnung: Ethocel Type N 22) und 37,5 g Polyethylenglykol 1500 (Handelsbezeichnung zum Beispiel Carbowax 1540) in 720 g Chloroform durch Besprühen in der Wirbelschicht-Apparatur in üblicher Weise überzogen.

50 mg der oben erhaltenen überzogenen Pellets werden in Hartgelatinekapseln der Größe 3 gefüllt.

Eine Hartgelatinekapsel enthält 4,4 mg Azelastinhydrochlorid in Retardzubereitung.

### Beispiel 4:

Die Herstellung der erfindungsgemäßen Darreichungsformen erfolgt durch Einbetten in Quellstoffe:
Folgende Substanzen werden gemischt: (Angaben in Gramm)

| | Rezeptur | | |
|---|---|---|---|
| | 1 | 2 | 3 |
| Azelastinhydrochlorid | 50 | 50 | 50 |
| Hydroxypropylmethylcellulose (= Methocel K 4 M) | 480 | 192 | 96 |
| Lactose | 660 | 948 | 1044 |
| Magnesiumstearat | 10 | 10 | 10 |

Die Mischungen werden auf einer Tablettenpresse zu Plantabletten mit einem Gewicht von 120 mg und einem Durchmesser von 6 mm gepresst.

Es beträgt die

| | | | |
|---|---|---|---|
| Dicke mm | 3,25 | 3,15 | 3,05 |
| Bruchfestigkeit (N) (Heberlein Bruchfestigkeitstester) | 47 | 48 | 50 |

Eine Tablette enthält 5 mg Azelastin HCl.
Die Wirkstofffreisetzung in der Apparatur der USP XXI (Dissolutiontester Apparatus 2, Lösungsmedium: 500 ml 0,1 N HCl,
Umdrehungszahl: 120 Upm) ist folgende: (Angabe der Wirkstofffreisetzung in %)

| | Rezeptur | | |
|---|---|---|---|
| | 1 | 2 | 3 |
| Nach 5 Min. | 4 | 31 | 50 |
| 60 Min. | 20 | 76 | 100 |
| 120 Min. | 36 | 96 | |
| 180 Min. | 55 | | |
| 240 Min. | 74 | | |
| 300 Min. | 86 | | |
| 360 Min. | 93 | | |

### Beispiel 5

100 g Azelastinhydrochlorid, 200 g Weinsäure, 500 g Milchzucker und 700 g mikrokristalline Cellulose werden gemischt und mit etwa 700 g gereinigtem Wasser angeteigt. Die feuchte Masse wird durch ein Lochblech mit einem Lochdurchmesser von 1 mm gepreßt und die entstehenden Stränge durch Behandlung auf einer Spheronizer-Scheibe in üblicher Weise zerteilt und ausgerundet. Die erhaltenen Pellets werden getrocknet und gesiebt.

1000 g Pellets der Siebfraktion 800 bis 1250 µm werden mit einer Suspension besprüht, die folgendermaßen hergestellt wird:

In 190 g gereinigtem Wasser werden 0,6 g Polysorbat 80 gelöst und in der Lösung 40 g Triethylcitrat emulgiert. Zu der erhaltenen Emulsion werden 800 g einer 30%igen wässrigen Dispersion eines Copolymerisates aus Acryl- und Methacrylsäureestern mit einem geringen Gehalt an Trimethylammoniumethacrylatchlorid (= Eudragit® RS 30 D) zugegeben und etwa 10 Minuten lang gerührt.

In 860 g gereinigtem Wasser werden 109,2 g Talkum und 0,2 g Silikonantischaumöl (Simethicone) suspendiert. Diese Suspension wird in die oben erhaltene Dispersion eingerührt.

Der Auftrag der damit erhaltenen Lackier-Suspension auf die Pellets geschieht in üblicher Weise, zum Beispiel unter Verwendung eines Wirbelschichtsprühgranulators bei einer Zulufttemperatur von 40-50^{o} C und einer Ablufttemperatur von maximal 40^{o} C. Die Trocknung der Pellets erfolgt unter den gleichen Bedingungen.

Es wird so viel von oben genannter Lackier-Suspension aufgesprüht, bis das Gesamtgewicht der getrockneten Pellets 1042 g beträgt.

Die lackierten Pellets werden in einer Menge von 78,1 mg in Hartgelatinekapseln der Größe 3 gefüllt. Eine Hartgelatinekapsel enthält 5 mg Azelastinhydrochlorid in Retardzubereitung. Die Freisetzung des Wirkstoffes aus einer Kapsel in der Apparatur der USP XXI (Dissolutiontester, Apparatus 2, Dissolution Medium: 500 ml 0,1 N HCl, Rotation Speed: 120 rpm) beträgt:
Nach 1 Stunde 3,0 mg ≙ 60 %
Nach 2 Stunden 4,5 mg ≙ 90 %
Die Wirkstofffreisetzung ist also 3 mg pro Stunde.

### Beispiel 6

Es wird gearbeitet wie in Beispiel 5 angegeben, nur wird von der in Beispiel 5 genannten Lackier-Suspensionen so viel auf die Pellets gesprüht, bis das Gesamtgewicht der getrockneten Pellets 1127 g beträgt. Sodann werden die lackierten Pellets zu
84,5 mg in Hartgelatinekapseln der Größe 3 gefüllt. Eine Hartgelatinekapsel enthält 5 mg Azelastinhydrochlorid in Retardzubereitung. Die Freisetzung des Wirkstoffes aus einer Kapsel in der Apparatur der USP XXI beträgt (Bedingungen bei der Prüfung wie in Beispiel 5):
Nach 1 Stunde 0,25 mg ≙ 5 %
Nach 2 Stunden 0,50 mg ≙ 10 %
Die Wirkstofffreisetzung ist also 0,25 mg pro Stunde.

### Beispiel 7

Es wird gearbeitet wie in Beispiel 6 angegeben.

Werden von den in Beispiel 6 erhaltenen Pellets 16,9 mg in Hartgelatinekapseln der Größe 3 gefüllt, so enthält eine Hartgelatinekapsel 1 mg Azelastinhydrochlorid in Retardzubereitung. Die Freisetzung des Wirkstoffes aus einer Kapsel in der Apparatur der USP XXI (Bedingungen bei der Prüfung wie in Beispiel 5) beträgt:
Nach 1 Stunde 0,05 mg ≙ 5 %
Nach 2 Stunden 0,10 mg ≙ 10 %
Die Wirkstofffreisetzung ist also 0,05 mg pro Stunde.

### Beispiel 8

Kapseln mit 6 mg Azelastin oder Suspensionen mit 6 mg Azelastin in 5 ml, jeweils gebunden an stark sauren Kationenaustauscher.

8,48 g Azelastinhydrochlorid werden in 4 Litern gereinigtem Wasser gelöst. In die Lösung werden 71,5 g Styrolsulfonsäure-Divinylbenzol-Copolymerisat (Vernetzungsgrad 8 %) (Handelsprodukt: Zum Beispiel Amberlite® IR 120) suspendiert und die Suspension 3 Stunden lang gerührt. Die Suspension wird danach durch eine Glasfilternutsche Filtriert und der erhaltene Filterkuchen zweimal mit jeweils 300 ml gereinigtem Wasser gewaschen und das Waschwasser gut abgesaugt.

5 g Gelatine (isoelektrischer Punkt 6 - 7,6; Molekulargewicht 25 000 - 35 000) (Handelsprodukt: Gelita® Collagel, Deutsche Gelatinefabriken, Eberbach/Neckar) werden in einer Lösung von 5 g 1-normaler Salzsäure in 800 g gereinigtem Wasser in einem Becherglas gelöst. Der oben erhaltene Filterkuchen wird in der Lösung suspendiert und die Suspension 1 Stunde lang gerührt. Danach wird die Suspension durch eine Glasfilternutsche filtriert und der erhaltene Filterkuchen zweimal mit jeweils 200 ml gereinigtem Wasser gewaschen und das Waschwasser abgesaugt.

Der Filterkuchen wird bei 60^{o}C getrocknet. Das getrocknete Produkt wird zu 62 mg in Hartgelatinekapseln der Größe 4 abgefüllt.

Eine Hartgelatinekapsel enthält 6 mg Azelastin, gebunden an stark sauren Kationenaustauscher.

Ein Saft, der 6 mg Azelastin, gebunden an Ionenaustauscher in 5 ml enthält, wird folgendermaßen erhalten:
7,4 kg gereinigtes Wasser werden auf 90 - 95^{o}C erwärmt und darin 0,002 kg Propyl-4-hydroxybenzoat und 0,013 kg Methyl-4-hydroxybenzoat gelöst. In der auf 70^{o}C abgekühlten Lösung werden 0,020 kg Hydroxyethylcellulose (durchschnittlicher Polymerisationsgrad: 250) und 3,0 kg Saccharose gelöst.

Nach Abkühlen auf 25^{o}C werden 3 g Himbeer-Aroma und 0,2 kg Modifizierte Stärke (Starch 1500®/Colorcon) unter Rühren gelöst beziehungsweise suspendiert. In die Suspension werden 124 g des getrokkneten, mit Azelastin beladenen Ionenaustauschers eingerührt. Die Suspension wird anschließend mit gereinigtem Wasser auf 11,0 kg (entsprechend 10 Liter) aufgefüllt.

Die Freisetzung des Wirkstoffs aus einer Kapsel oder 5 ml Suspension in der Apparatus der USP XXI (Dissolutiontester, Apparatus 2, Dissolution Medium 500 ml Natriumchlorid 0,9 %, Rotation Speed: 100 rpm) beträgt:

| | |
|---|---|
| Nach 1 Stunde | 25 % |
| 2 Stunden | 40 % |
| 3 Stunden | 50 % |
| 4 Stunden | 58 % |
| 5 Stunden | 65 % |
| 6 Stunden | 69 % |
| 7 Stunden | 72 % |
| 8 Stunden | 75 %. |

Dabei wird jede Stunde das Dissolution Medium erneuert; die erhaltenen Werte für die Freisetzung werden aufaddiert.

## Patentansprüche

1. Azelastin enthaltende Arzneimittelzubereltungen mit kontrollierter Wirkstofffreigabe unter Verwendung einer Retardkomponente, wobei auf einen Gewichts-Teil Azelastin (bezogen auf die Base) 0,004 bis 800 Teile Retardkomponente kommen und das Azelastin auch in Form seiner physiologisch verträglichen Salze vorliegen kann und die Freisetzungsgeschwindigkeit von Azelastin zwischen 0,05 und 5 mg pro Stunde beträgt, wobei die Freisetzungsgeschwindigkeit bestimmt wird in einer wässrigen Prüflösung vom pH 1,0 und/oder pH 6,8.

2. Arzneimittelzubereitung nach Anspruch 1,
**dadurch gekennzeichnet**,
daß der Wirkstoff Azelastin oder seine physiologisch verträglichen Salze gegebenenfalls unter Zusatz von sonstigen üblichen Hilfs- und Zusatzstoffen zur kontrollierten Freisetzung
a) mit einer oder mehreren Retardkomponenten umhüllt ist, oder
b) an einen Kationenaustauscher gebunden ist, oder
c) mit einer oder mehreren osmotisch aktiven Substanzen versetzt und mit einer semipermeablen Membran umhüllt ist und ein Loch gebohrt ist, oder
d) in eine oder mehrere Substanzen aus den Gruppen der verdaulichen Fette, der unverdaulichen Fette beziehungsweise fettähnlichen Substanzen, Polymeren oder Quellstoffe eingebettet oder an diese Substanzen gebunden ist.

3. Arzneimittelzubereitung nach Anspruch 1,
**dadurch gekennzeichnet**,
daß die Darreichungsformen für die orale Anwendung 0,1 bis 50 mg, für die parenteral Anwendung 0,1 bis 500 mg und für die dermale Anwendung 5 bis 5000 mg Azelastin-Wirkstoff enthalten.

4. Verfahren zur Herstellung einer den Wirkstoff Azelastin enthaltenden Arzneimittelzubereitung mit kontrollierter Wirkstofffreigabe,
**dadurch gekennzeichnet**,
daß man einen Gewichtsteil Azelastin (bezogen auf die Base), wobei das Azelastin auch in Form seiner physiologisch verträglichen Salze vorliegen kann, mit 0,004 bis 800 Gewichtsteilen einer Retardkomponente und gegebenenfalls weiteren üblichen pharmazeutischen Hilfs- und Zusatzstoffen verar-beitet und eine Freisetzungsgeschwindigkeit von 0,05 bis 5 mg Azelastin/Stunde einstellt.

5. Verfahren zur Herstellung einer Arzneimittelzubereitung nach Anspruch 4,
**dadurch gekennzeichnet**,
daß der Wirkstoff Azelastin oder seine physiologisch verträglichen Salze
a) mit einer oder mehreren Retardkomponenten umhüllt wird, oder
b) an einen Kationenaustauscher gebunden wird, oder
c) mit einer oder mehreren osmotisch aktiven Substanzen versetzt und mit einer semi-permeablen Membran umhüllt wird, wobei in die Membran ein Loch gebohrt wird, oder
d) an eine oder mehrere Substanzen aus den Gruppen der verdaulichen Fette, der unverdaulichen Fette beziehungsweise fettähnlichen Substanzen, Polymeren oder Quellstoffe eingebettet oder an diese Substanzen gebunden wird.
wobei zusätzlich bei den zuvor angegebenen Arbeitsweisen auch noch weitere übliche Hilfs- und Zusatzstoffe mitverwendet werden können.

6. Verfahren zur Herstellung einer Arzneimittelzubereitung nach Anspruch 4 und/oder 5,
**dadurch gekennzeichnet**,
daß die Darreichungsformen für die orale Anwendung 0,1 bis 50 mg, bei für die parenteraler Anwendung 0,1 bis 500 mg und für die dermale Anwendung 5 bis 5000 mg Azelastin-Wirkstoff enthalten.

## Claims

**1.** Azelastine-containing medicinal preparations with controlled release of the active principle using a retard component, 0.004 to 800 parts of the retard component being used to one part by weight azelastine (based on the base), the azelastine optionally being present in the form of its physiologically acceptable salts and the release rate of the azelastine being between 0.05 and 5 mg per hour, the release rate being determined in an aqueous test solution of pH 1.0 and/or pH 6.8.

**2.** A medicinal preparation as claimed in claim 1, characterized in that the active principle azelastine or its physiologically acceptable salts is/are
a) coated with one or more retard components or
b) fixed to a cation exchanger or
c) mixed with one or more osmotically active substances and covered with a semipermeable membrane and a hole is drilled or
d) is/are encapsulated in, or fixed to, one or more substances from the group consisting of digestible fats, indigestible fats or fat-like substances, polymers or swellable substances,
optionally with addition of other typical auxiliaries and additives for controlled release.

**2.** A medicinal preparation as claimed in claim 1, characterized in that the formulations for oral administration contain 0.1 to 50 mg, those for parenteral administration 0.1 to 500 mg and those for dermal application 5 to 5000 mg azelastine.

**4.** A process for the production of a medicinal preparation containing the active principle azelastine with controlled release thereof, characterized in that one part by weight azelastine (based on the base) is processed with 0.004 to 800 parts by weight of a retard component and optionally other typical pharmaceutical auxiliaries and additives and a release rate of 0.05 to 5 mg azelastine/hour is adjusted.

**5.** A process for the production of a medicinal preparation as claimed in claim 4, characterized in that the active principle azelastine or its physiologically acceptable salts is/are
a) coated with one or more retard components or
b) fixed to a cation exchanger or
c) mixed with one or more osmotically active substances and covered with a semipermeable membrane and a hole is drilled or
d) is/are encapsulated in, or fixed to, one or more substances from the group consisting of digestible fats, indigestible fats or fat-like substances, polymers or swellable substances,
other typical auxiliaries and additives optionally being additionally used in the steps mentioned above.

**6.** A process for the production of a medicinal preparation as claimed in claim 4 and/or 5, characterized in that the formulations for oral administration contain 0.1 to 50 mg, those for parenteral administration 0.1 to 500 mg and those for dermal application 5 to 5000 mg azelastine.

## Revendications

1. Compositions medicamenteuses contenant de l'azélastine a libération contrôlée de la substance active par utilisation d'un composant retard, dans lesquelles il est contenu, pour l partie en poids d'azélastine (rapportee à la base), de 0,004 à 800 parties de composant retard, l'azélastine peut aussi se présenter sous forme de ses sels acceptables physiologiquement et la vitesse de libération de l'azélastine est comprise entre 0,05 et 5 mg par heure, cette vitesse de liberation etant determinée dans une solution aqueuse expérimentale ayant un pH de 1,0 et/ou un pH de 6,8.

2. Composition medicamenteuse selon la revendication 1, caracterisée en ce que, pour leur libération controlée, la substance active azélastine ou ses sels acceptables physiologiquement, auxquels sont éventuellement ajoutes d'autres adjuvants et additifs usuels,
a) sont enrobés d'un ou de plusieurs composants retard ou
b) sont fixes sur un echangeur de cations ou
c) sont additionnès d'une ou de plusieurs substances à activité osmotique, enrobés d'une membrane semipermeable dans laquelle est percé un trou ou
d) sont incorpores dans une ou plusieurs substances du groupe des graisses digestibles, des graisses non digestibles ou des substances semblables aux graisses, dans des polymères ou dans des matières gonflantes, ou sont fixes sur ces substances.

3. Composition médicamenteuse selon la revendication 1, caractérisée en ce que les formes d'administration contiennent de 0,1 à 50 mg de substance active azélastine pour l'usage par voie orale, de 0,1 à 500 mg pour l'usage par voie parentérale et de 5 à 5000 mg pour l'usage par voie cutanée.

4. Procédé de préparation d'une composition médicamenteuse contenant la substance active azélastine, à libération contrôlée de la substance active, caractérisé en ce qu'on travaille 1 partie en poids d'azélastine (rapportée à la base), l'azélastine pouvant aussi se présenter sous forme de ses sels acceptables physiologiquement, avec 0,004 à 800 parties en poids d'un composant retard et éventuellement avec d'autres adjuvants et additifs pharmaceutiques usuels, et on règle une vitesse de libération de 0,05 à 5 mg d'azélastine/heure.

5. Procédé de preparation d'une composition médicamenteuse selon la revendication 4, caractérisé en ce que la substance active azélastine ou ses sels acceptables physiologiquement
a) sont enrobés d'un ou de plusieurs composants retard ou
b) sont fixes sur un echangeur de cations ou
c) sont additionnés d'une ou de plusieurs substances à activité osmotique et enrobés d'une membrane semiperméable, un trou etant percé dans la membrane ou
d) sont incorpores dans une ou plusieurs substances du groupe des graisses digestibles, des graisses non digestibles ou des substances semblables aux graisses, dans des polymeres ou dans des matieres gonflantes, ou sont fixes sur ces substances,
d'autres adjuvants et additifs usuels pouvant être en plus utilisés simultanément dans les modes opératoires susmentionnés.

6. Procedé de preparation d'une composition medicamenteuse selon les revendications 4 et/ou 5, caractérisé en ce que les formes d'administration contiennent de 0,1 à 50 mg de substance active azélastine pour l'usage par voie orale, de 0,1 à 500 mg pour l'usage par voie parentérale et de 5 à 5000 mg pour l'usage par voie cutanée.
